# EUROPÄISCHE PATENTANMELDUNG

(11) **EP 2 042 156 A1**
(43) Veröffentlichungstag der Anmeldung: **01.04.2009**
(21) Anmeldenummer: 07120415.0
(22) Anmeldetag: 09.11.2007
(51) Int. Cl.: A61K 8/64, A61Q 1/14, A61Q 19/10, C11D 3/38, C11D 11/00, A61Q 5/02

(54) **Verfahren zum Entfernen von wasserunlöslichen Substanzen von Substratoberflächen**

(30) Priorität: 28.09.2007 EP 07117555
(71) Anmelder: BASF SE, 67056 Ludwigshafen (DE)
(72) Erfinder: Barg, Heiko, Dr., 67346 Speyer (DE); Subkowski, Thomas, Dr., 68526 Ladenburg (DE); Karos, Marvin, Dr., 68723 Schwetzingen (DE); Bollschweiler, Claus, Dr., 69118 Heidelberg (DE)

(57) **Zusammenfassung**

Die Erfindung betrifft ein Verfahren zum Entfernen von wasserunlöslichen Substanzen von Substratoberflächen mittels eines Trägers enthaltend Hydrophobin.

## Beschreibung

Die Erfindung betrifft ein Verfahren zum Entfernen von wasserunlöslichen Substanzen von Substratoberflächen mittels eines Trägers enthaltend Hydrophobin.
Verfahren zum Entfernen von wasserunlöslichen Substanzen von Substraten mittels eines Trägers aus dem täglichen Leben sind insbesondere aus dem Haushalt bekannt. Die meisten dieser Putz-, Reinigungs- oder Sprühmittel sowie auch kosmetische Mittel zum Entfernen von Make-up oder Hautreinigung beruhen auf Lösungen, die organische Lösungsmittel, zum Beispiel Alkohol, und Emulgatoren oder Detergenzien enthalten, um die wasserunlöslichen Substanzen auf den Substraten zu lösen. Ferner können solche Mittel auch Partikel enthalten, wie Scheuermittel oder Peeling-Präparate, die durch mechanische Reibung eine Reinigungswirkung entfalten. Der Großteil dieser Zubereitungen wird auf das Substrat aufgetragen und anschließend mit einem Tuch, aus Textilien, Papier oder Watte, aufgenommen und so das Substrat gereinigt.
In den letzten Jahren werden vermehrt auch feuchte Materialien angeboten, welche die entsprechenden Zubereitungen zur Reinigung der Substrate enthalten. Insbesondere im Bereich der Kosmetik sind feuchte Reinigungstücher oder so genannte Wattepads mit den entsprechenden Zubereitungen bekannt.

Die Verwendung von Hydrophobin in kosmetischen Zubereitungen ist an sich bekannt. Hydrophobine sind kleine Proteine von etwa 100 bis 150 Aminosäuren, welche in filamentösen Pilzen, beispielsweise Schizophyllum commune, vorkommen. Sie weisen in aller Regel 8 Cystein-Einheiten auf. Hydrophobine können aus natürlichen Quellen isoliert werden, können aber auch mittels gentechnischer Verfahren gewonnen werden, wie beispielsweise von WO 2006/082251 oder WO 2006/131564 offenbart.

Die US 20030217419A1 beschreibt die Verwendung des Hydrophobins SC3 aus Schizophyllum commune für kosmetische Zubereitungen zur Behandlung von Therapie-Materialien. Dabei bilden sich kosmetische Depots die mehreren Wäschen mit Shampoo widerstehen.

Im Stand der Technik ist die Verwendung von Hydrophobinen auch für andere Anwendungen vorgeschlagen worden.

WO 96/41882 schlägt die Verwendung von Hydrophobinen als Emulgatoren, Verdicker, oberflächenaktive Substanzen, zum Hydrophilieren hydrophober Oberflächen, zur Verbesserung der Wasserbeständigkeit hydrophiler Substrate, zur Herstellung von Öl-in-Wasser-Emulsionen oder von Wasser-in-Öl-Emulsionen vor. Weiterhin werden pharmazeutische Anwendungen wie die Herstellung von Salben oder Cremes sowie kosmetische Anwendungen z.B. zum Hautschutz oder für die Herstellung von Haarshampoos oder Haarspülungen vorgeschlagen. EP 1 252 516 offenbart die Beschichtung verschiedener Substrate mit einer Hydrophobine enthaltenden Lösung bei einer Temperatur von 30 bis 80°C. Weiterhin wurde beispielsweise die Verwendung als Demulgator (WO 2006/103251), als Verdunstungsverzögerer (WO 2006/128877) oder Verschmutzungsinhibitor (WO 2006/103215) vorgeschlagen.

Eine Aufgabe der vorliegenden Erfindung war es, ein Verfahren zum Entfernen von wasserunlöslichen Substanzen von Substratoberflächen zur Verfügung zu stellen, das vielseitig einsetzbar ist. Darüber hinaus sollte das Verfahren schonend für die Substratoberfläche sein. Zusätzlich sollte das Verfahren einfach sein und das Entfernen der wasserunlöslichen Substanzen mit wenigen Handgriffen erlauben.
Desweiteren war es eine Aufgabe der Erfindung, eine neue Verwendung für Hydrophobin bereitzustellen.

Die Aufgaben werden durch das eingangs beschriebene Verfahren sowie die weiteren Gegenstände der vorliegenden Erfindung gelöst. Ein weiterer Gegenstand der vorliegenden Erfindung betrifft kosmetische Verfahren zum Entfernen von Sebum und/oder Schminke, bei dem man die Haut- und/oder Haaroberfläche mit einem Träger, der Hydrophobin enthält, behandelt. Darüber hinaus ist die Verwendung eines Trägers, der Hydrophobin enthält, zum Entfernen von wasserunlöslichen Substanzen Gegenstand der vorliegenden Erfindung. Desweiteren umfasst die vorliegende Erfindung die Verwendung von Hydrophobin zur Herstellung eines kosmetischen Mittels zum Entfernen von Sebum sowie eines kosmetischen Mittels zur Prophylaxe und/oder Behandlung von Akne und/oder Keratosis pilaris. Besondere Ausführungsformen der Erfindung sind den Ansprüchen, der Beschreibung und den Beispielen zu entnehmen. Ferner umfasst die Erfindung auch Kombinationen bevorzugter Ausführungsformen.

Im erfindungsgemäßen Verfahren werden Träger eingesetzt, die Hydrophobin enthalten. Darüber hinaus wird Hydrophobin erfindungsgemäß zur Herstellung von kosmetischen Mitteln verwendet.
Hydrophobine sind obenflächenaktive Polypeptide. Sie können aus natürlichen Quellen isoliert werden, können aber auch mittels gentechnischer Verfahren gewonnen werden.

Prinzipiell sind sowohl Hydrophobine der einen wie der anderen Art geeignet.

Unter "Hydrophobin" bzw. "Hydrophobine" können Polypeptide der allgemeinen Formel (I)

Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)

verstanden werden, wobei X für jede der 20 natürlich vorkommenden Aminosäuren (Phe, Leu, Ser, Tyr, Cys, Trp, Pro, His, Gln, Arg, Ile Met, Thr, Asn, Lys, Val, Ala, Asp, Glu, Gly) stehen kann. Dabei können die Reste X jeweils gleich oder verschieden sein. Hierbei stellen die bei X stehenden Indizes jeweils die Anzahl der Aminosäuren in der jeweiligen Teilsequenz X dar, C steht für Cystein, Alanin, Serin, Glycin, Methionin oder Threonin, wobei mindestens vier der mit C benannten Reste für Cystein stehen, und die Indizes n und m stehen unabhängig voneinander für natürliche Zahlen. Im Allgemeinen sind weder m noch n Null, sondern betragen in der Regel 1 oder mehr. Beispielsweise können m und n unabhängig voneinander von 1 bis 500 betragen. Bevorzugt sind m und n unabhängig voneinander von 15 bis 300. Die mit C¹ bis C⁸ benannten Aminosäuren sind bevorzugt Cysteine; sie können aber auch durch andere Aminosäuren ähnlicher Raumerfüllung, bevorzugt durch Alanin, Serin, Threonin, Methionin oder Glycin ersetzt werden. Allerdings sollen mindestens vier, bevorzugt mindestens fünf, besonders bevorzugt mindestens sechs und insbesondere mindestens sieben der Positionen C¹ bis C⁸ aus Cysteinen bestehen. Cysteine können in den erfindungsgemäßen Proteinen entweder reduziert vorliegen oder miteinander Disulfidbrücken ausbilden. Besonders bevorzugt ist die intramolekulare Ausbildung von C-C Brücken, insbesondere die mit mindestens einer, bevorzugt zwei, besonders bevorzugt drei und ganz besonders bevorzugt vier intramolekularen Disulfidbrücken. Bei dem oben beschriebenen Austausch von Cysteinen durch Aminosäuren ähnlicher Raumerfüllung werden vorteilhaft solche C-Positionen paarweise ausgetauscht, die intramolekulare Disulfidbrücken untereinander ausbilden können.
Falls in den mit X bezeichneten Positionen auch Cysteine, Serine, Alanine, Glycine, Methionine oder Threonine verwendet werden, kann sich die Nummerierung der einzelnen C-Positionen in den allgemeinen Formeln entsprechend verändern.
Bevorzugt werden Hydrophobine der allgemeinen Formel (II)

Xₙ-C¹-X₃₋₂₅-C²-X₀₋₂-C³-X₅₋₅₀-C⁴-X₂₋₃₅-C⁵-X₂₋₁₅-C⁶-X₀₋₂-C⁷-X₃₋₃₅-C⁸-Xₘ (II)

zur Ausführung der vorliegenden Erfindung eingesetzt, wobei X und C die obige Bedeutung sowie die bei X stehenden Indizes die obige Bedeutung haben, die Indizes n und m für natürliche Zahlen. Im Allgemeinen sind weder m noch n Null, sondern betragen in der Regel 1 oder mehr. Beispielsweise können m und n unabhängig voneinander von 1 bis 500 betragen. Bevorzugt sind m und n unabhängig voneinander von 15 bis 300 und bei mindestens sechs der mit C benannten Reste handelt es sich um Cystein. Besonders bevorzugt handelt es sich bei allen Resten C um Cystein.

Besonders bevorzugt werden Hydrophobine der allgemeinen Formel (III)

Xₙ-C¹-X₅₋₉-C²-C³-X₁₁₋₃₉-C⁴-X₂₋₂₃-C⁵-X₅₋₉-C⁶-C⁷-X₆₋₁₈-C⁸-Xₘ (III)

eingesetzt, wobei X und C die obige Bedeutung sowie die bei X stehenden Indizes die obige Bedeutung haben. Inbesondere stehen die Indizes n und m für natürliche Zahlen von 1 bis 200. Im Allgemeinen handelt es sich bei mindestens sechs der mit C benannten Reste um Cystein . Besonders bevorzugt handelt es sich bei allen Resten C um Cystein.
Bei den Gruppen Xₙ und Xₘ kann es sich um Peptidsequenzen handeln, die natürlicherweise mit den anderen Bestandteilen des Hydrophobins verknüpft sind. Es kann sich aber auch bei einem oder bei beiden Gruppen um Peptidsequenzen handeln, die natürlicherweise nicht mit den anderen Bestandteilen des Hydrophobins verknüpft sind. Darunter sind auch solche Gruppen Xₙ und/oder Xₘ zu verstehen, bei denen eine natürlicherweise im Protein vorkommende Peptidsequenz durch eine nicht natürlicherweise im Protein vorkommende Peptidsequenz verlängert ist.
Die Gruppe Xₙ und/oder Xₘ kann ganz oder teilweise Peptidsequenzen enthalten, die nicht natürlicherweise im Protein vorkommen. Die nicht natürlicherweise im Protein vorkommenden Peptidsequenzen, aus denen die Gruppe Xₙ und/oder Xₘ teilweise oder ganz bestehen kann, soll im Folgenden auch als Fusionspartner bezeichnet werden. Diese Fusionspartner sind in der Regel mindestens 20, bevorzugt mindestens 35 Aminosäuren lang. Es kann sich beispielsweise um Sequenzen aus von 20 bis 500, bevorzugt von 30 bis 400 und besonders bevorzugt von 35 bis 100 Aminosäuren handeln.
Fusionspartner sind beispielsweise in WO 2006/082251, WO 2006/082253 und WO 2006/131564 offenbart worden.
Der Fusionspartner kann aus einer Vielzahl von Proteinen ausgewählt werden. Es kann nur ein einziger Fusionspartner mit dem Rest des Polypeptids verknüpft sein, oder es können auch mehrere Fusionspartner mit dem Rest des Polypeptids verknüpft werden. Es können aber auch beispielsweise zwei Fusionspartner an den Positionen Xₙ oder Xₘ mit dem Rest des Polypeptids verknüpft werden.
Besonders geeignete Fusionspartner sind Proteine, die natürlicherweise in Mikroorganismen, insbesondere in Escherichia coli oder Bacillus subtilis vorkommen. Beispiele für solche Fusionspartner sind die Sequenzen yaad (SEQ ID NO: 16 in WO 2006/082251), yaae (SEQ ID NO:18 in WO 2006/082251), Ubiquitin und Thioredoxin. Gut geeignet sind auch Fragmente oder Derivate dieser genannten Sequenzen, die nur einen Teil, beispielsweise von 70 bis 99 %, bevorzugt von 5 bis 50 %, und besonders bevorzugt von 10 bis 40 % der genannten Sequenzen umfassen, oder bei denen einzelne Aminosäuren, bzw. Nukleotide gegenüber der genannten Sequenz verändert sind, wobei sich die Prozentangaben jeweils auf die Anzahl der Aminosäuren bezieht.

In einer weiterhin bevorzugten Ausführungsform weist das Hydrophobin neben dem genannten Fusionspartner als eine der Gruppen Xₙ oder Xₘ oder als terminaler Bestandteil einer solchen Gruppe noch eine sogenannte Affinitätsdomäne (affinity tag /affinity tail) auf. Hierbei handelt es sich in prinzipiell bekannter Art und Weise um Ankergruppen, welche mit bestimmten komplementären Gruppen Wechselwirken können und der leichteren Aufarbeitung und Reinigung der Proteine dienen können. Beispiele derartiger Affinitätsdomänen umfassen (His)ₖ-, (Arg)ₖ-, (Asp)ₖ-, (Phe)ₖ- oder (Cys)ₖ-Gruppen, wobei k im allgemeinen für eine natürliche Zahl von 1 bis 10 steht. Bevorzugt kann es sich um eine (His)ₖ-Gruppe handeln, wobei k für vier bis sechs steht. Hierbei kann die Gruppe Xₙ und/oder Xₘ ausschließlich aus einer derartigen Affinitätsdomäne bestehen oder aber aus natürlicherweise oder nicht natürlicherweise mit dem Rest des Polypeptids verknüpften Aminosäuren und einer derartigen Affinitätsdomäne.
In einer weiteren der bevorzugten Ausführungsformen können Hydrophobine auch noch in ihrer Polypeptidsequenz modifiziert sein, beispielsweise durch Glycosilierung, Acetylierung oder auch durch chemische Quervernetzung beispielsweise mit Glutardialdehyd.
Eine biologische Eigenschaft der eingesetzten bzw. verwendeten Hydrophobine ist die Änderung von Oberflächeneigenschaften, wenn die Oberflächen z.B. die eines Trägers mit den Proteinen beschichtet werden. Die Änderung der Oberflächeneigenschaften lässt sich experimentell beispielsweise dadurch bestimmen, dass der Kontaktwinkel eines Wassertropfens vor und nach der Beschichtung der Oberfläche mit den Proteinen gemessen wird und die Differenz der beiden Messungen ermittelt wird.
Die Durchführung von Kontaktwinkelmessungen ist dem Fachmann prinzipiell bekannt. Die Messungen werden bei Raumtemperatur mit einem Wassertropfen von 5 µl und unter Verwendung von Glasplättchen als Substrat durchgeführt. Die genauen experimentellen Bedingungen für eine beispielhaft geeignete Methode zur Messung des Kontaktwinkels sind im experimentellen Teil dargestellt. Unter den dort genannten Bedingungen können die verwendeten Hydrophobine den Kontaktwinkel vergrößern. So können die Hydrophobine den Kontaktwinkel beispielsweise um mindestens 20°, bevorzugt mindestens 25°, besonders bevorzugt mindestens 30°; 40°,45° insbesondere 50° vergrößern, jeweils verglichen mit dem Kontaktwinkel eines gleich großen Wassertropfens mit der unbeschichteten Glasoberfläche verändern.
Besonders bevorzugte Hydrophobine zur Ausführung der vorliegenden Erfindung sind die Hydrophobine des Typs dewA, rodA, hypA, hypB, sc3, basf1, basf2. Diese Hydrophobine inklusive ihrer Sequenzen sind beispielsweise in WO 2006/82251 offenbart. Sofern nicht anders angegeben, beziehen sich die nachfolgend angegebenen Sequenzen auf in WO 2006/82251 offenbarten Sequenzen. Eine Übersichtstabelle mit den SEO-ID-Nummern befindet sich in WO 2006/82251 auf Seite 20.

Erfindungsgemäß insbesondere geeignet sind die Hydrophobine yaad-Xa-dewA-his (SEQ ID NO: 20), yaad-Xa-rodA-his (SEQ ID NO: 22) oder yaad-Xa-basf1-his (SEQ ID NO: 24) mit den in Klammern angegebenen Polypeptidsequenzen sowie den dafür codierenden Nukleinsäuresequenzen, insbesondere den Sequenzen gemäß SEQ ID NO: 19, 21, 23. Besonders bevorzugt kann yaad-Xa-dewA-his (SEQ ID NO: 20) eingesetzt werden. Auch Proteine, die sich ausgehend von den in SEQ ID NO. 20, 22 oder 24 dargestellten Polypeptidsequenzen durch Austausch, Insertion oder Deletion von mindestens einer, bis hin zu zehn, bevorzugt fünf Aminosäuren, besonders bevorzugt 5% aller Aminosäuren ergeben, und die die biologische Eigenschaft der Ausgangsproteine noch zu mindestens 50% besitzen, sind besonders bevorzugte Ausführungsformen. Unter biologischer Eigenschaft der Proteine wird hierbei die bereits beschriebene Änderung des Kontaktwinkels verstanden.
Besonders zur Ausführung der vorliegenden Erfindung geeignete Hydrophobine sind von yaad-Xa-dewA-his (SEQ ID NO: 20), yaad-Xa-rodA-his (SEQ ID NO: 22) oder y-aad-Xa-basf1-his (SEQ ID NO: 24) durch Verkürzung des yaad-Fusionspartners abgeleitete Hydrophobine. Anstelle des vollständigen yaad-Fusionspartners (SEQ ID NO: 16) mit 294 Aminosäuren kann vorteilhaft ein verkürzter yaad-Rest eingesetzt werden. Der verkürzte Rest sollte aber zumindest 20, bevorzugt mindestens 35 Aminosäuren umfassen. Beispielsweise kann ein verkürzter Rest mit 20 bis 293, bevorzugt 25 bis 250, besonders bevorzugt 35 bis 150 und beispielsweise 35 bis 100 Aminosäuren eingesetzt werden. Ein Beispiel für ein derartiges Protein ist yaad40-Xa-dewA-his (SEQ ID NO: 26 in PCT/EP2006/064720), welches einen auf 40 Aminosäuren verkürzten yaad-Rest aufweist.
Eine Spaltstelle zwischen dem Fusionspartner bzw. Fusionspartnern und dem Rest des Polypeptids kann dazu genutzt werden, den Fusionspartner abzuspalten (beispielsweise durch BrCN-Spaltung an Methionin, Faktor Xa-, Enterokinase-, Thrombin-, TEV-Spaltung etc.).
Die erfindungsgemäß im Träger enthaltenen bzw. die zur Herstellung von kosmetischen Mitteln verwendeten Hydrophobine lassen sich chemisch durch bekannte Verfahren der Peptidsynthese, beispielsweise durch Festphasensynthese nach Merrifield herstellen.
Natürlich vorkommende Hydrophobine lassen sich aus natürlichen Quellen mittels geeigneter Methoden isolieren. Beispielhaft sei auf Wösten et. al., Eur. J Cell Bio. 63, 122-129 (1994) oder WO 96/41882 verwiesen.
Ein gentechnisches Herstellverfahren für Hydrophobine aus Talaromyces thermophilus, die keinen Fusionspartner enthalten, ist z.B. von US 2006/0040349 beschrieben.
Die Herstellung von Hydrophobinen, die einen Fusionspartner enthalten, kann bevorzugt durch gentechnische Verfahren erfolgen, bei denen eine für den Fusionspartner und eine für den Rest des Polypeptidscodierende Nukleinsäuresequenz, insbesondere DNA-Sequenz, so kombiniert werden, dass in einem Wirtsorganismus durch Genexpression der kombinierten Nukleinsäuresequenz das gewünschte Protein erzeugt wird. Ein derartiges Herstellverfahren beispielsweise ist von WO 2006/082251 oder WO 2006/082253 offenbart. Die Fusionspartner erleichtern die Herstellung der Hydrophobine erheblich. Hydrophobine, die einen Fusionspartner enthalten, werden bei den gentechnischen Verfahren mit deutlich besseren Ausbeuten produziert als Hydrophobine, die keinen Fusionspartner enthalten.
Die nach dem gentechnischen Verfahren von den Wirtsorganismen produzierten Hydrophobine können in prinzipiell bekannter Art und Weise aufgearbeitet und mittels bekannter chromatographischer Methoden gereinigt werden.
Im Allgemeinen werden gereinigte Hydrophobine zur Ausführung der Erfindung genutzt.
In einer bevorzugten Ausführungsform kann das in WO 2006/082253, Seiten 11/12 offenbarte, vereinfachte Aufarbeitungs- und Reinigungsverfahren eingesetzt werden.
Hierzu werden die fermentierten Zellen zunächst aus der Fermetationsbrühe abgetrennt, aufgeschlossen und die Zelltrümmer von den Einschlusskörpern (inclusion bodies) getrennt. Letzteres kann vorteilhaft durch Zentrifugieren erfolgen. Schließlich können die Einschlusskörper, beispielsweise durch Säuren, Basen und/oder Detergentien in prinzipiell bekannter Art und Weise aufgeschlossen werden, um die Hydrophobine freizusetzen. Die Einschlusskörper mit den erfindungsgemäß verwendeten Hydrophobinen können in der Regel schon unter Verwendung von 0,1 m NaOH innerhalb von ca. 1 h vollständig gelöst werden.
Die erhaltenen Lösungen können gegebenenfalls nach Einstellen des gewünschten pH-Wertes- ohne weitere Reinigung zur Ausführung dieser Erfindung eingesetzt werden. Die Hydrophobine können aus den Lösungen aber auch als Feststoff isoliert werden. Bevorzugt kann die Isolierung mittels Sprühgranulieren oder Sprühtrocknen erfolgen, wie in WO 2006/082253, Seite 12 beschrieben. Die nach dem vereinfachten Aufarbeitungs- und Reinigungsverfahren erhaltenen Produkte umfassen neben Resten von Zelltrümmern in der Regel ca. 80 bis 90 Gew. % Proteine. Die Menge an Hydrophobinen beträgt je nach Fermentationsbedingungen in der Regel von 30 bis 80 Gew. % bezüglich der Menge aller Proteine.
Die isolierten Hydrophobine enthaltenden Produkte können als Feststoffe gelagert werden.
Die Hydrophobine können als solche oder auch nach Abspaltung und Abtrennung des Fusionspartners zur Ausführung dieser Erfindung verwendet werden. Eine Spaltung nimmt man vorteilhaft nach der Isolierung der Einschlusskörper und deren Auflösung vor.

Erfindungsgemäß kann das Hydrophobin in einem Träger enthalten sein. Zudem kann der Träger ein Hydrophobin oder aber auch eine Zusammensetzung von verschiedenen Hydrophobinen enthalten, z.B. eine Zusammensetzung, die zwei oder drei Hydrophobine umfasst.

Im Allgemeinen ist der Träger weder flüssig noch gasförmig. In der Regel ist der Träger fest. Bevorzugt ist der Träger ein Material, das selbst eine Oberfläche aufweist, die nicht gänzlich glatt sondern unregelmäßig ist. Beispielsweise kann jene Oberfläche Vertiefungen aufweisen. Der Träger kann auch Hohlräume, Zellen und/oder Poren aufweisen. Der Träger kann aber auch aus einem faserförmigen Material oder einem Material sein, dass Fasern enthält. Bevorzugt ist der Träger ein Schaumstoff, Schwamm, Vliesstoff, Papier und/oder Watte.
Der Träger hat in einer Ausführungsform eine hydrophile Oberfläche.
In einer Ausführung der vorliegenden Erfindung werden Oberflächen als hydrophil bezeichnet, die Wasser nicht abstoßen. Hydrophile Oberflächen haben im Allgemeinen gegenüber Wasser Kontaktwinkel, die kleiner sind als 90°.
Ferner hat der Träger in einer besonderen Ausführung eine kleine Porengröße.
In einer Ausführung der vorliegenden Erfindung handelt es sich bei dem Träger um Schaumstoff. Schaumstoff ist ein Kunststoff, dessen Struktur durch viele Zellen (von Grundmaterial eingeschlossene Hohlräume, Poren) gebildet wird. Es eignen sich fast alle Kunststoffe zum Schäumen. In einer der Ausführungsformen werden Schaumstoffe bevorzugt, die sich für kosmetische Verwendungen eignen.

In einer besonderen Ausführung der vorliegenden Erfindung handelt es sich bei dem Träger um offenzellige Schaumstoffe, insbesondere auf Basis von Melamin-Formaldehyd-Harzen.
Herstellverfahren für Schaumstoffe auf Basis von Polyurethanen sind beispielsweise aus WO 2005/103107 oder WO 2006/008054 bekannt.

Herstellverfahren für Schaumstoffe auf Basis von Melamin-Formaldehyd-Harzen sind beispielsweise in EP-A 17 672 EP-A 37 470 sowie WO 01/94436 offenbart. Hiernach wird eine Mischung eines in einem wässrigen Medium gelösten oder dispergierten Melamin-Formaldehyd-Vorkondensates, eines Treibmittels, eines Dispergiermittels sowie eines Härters erwärmt, verschäumt und ausgehärtet. Das Erwärmen kann beispielsweise mithilfe von Heißluft, Wasserdampf oder Mikrowellenbetrahlung vorgenommen werden. Die Konzentration des Melamin/Formaldehyd-Vorkondensates in der Mischung beträgt in der Regel von 55 bis 85 Gew. %, bevorzugt von 63 bis 80 Gew.%. Die Rohdichte des offenzelligen Schaumstoffes auf Basis von Melamin-Formaldehyd-Harzen liegt in der Regel im Bereich von 3 bis 100 kg/m³, bevorzugt im Bereich von 5 bis 20 kg/m³. Der Begriff "Rohdichte" bezeichnet in prinzipiell bekannter Art und Weise die Dichte des Schaustoffes inklusive des Porenvolumens. Die Zellzahl liegt üblicherweise im Bereich von 50 bis 300 Zellen/25 mm. Die durchschnittliche Porengröße liegt üblicherweise im Bereich von 100 bis 250 µm (Meßmethode: systematische NMRanalyse mit molekularen Sonden oder Elektronenmikroskopie). Die Zugfestigkeit liegt bevorzugt im Bereich von 100 bis 150 kPa und die Bruchdehnung im Bereich von 8 bis 20% (Meßmethode für Zugfestigkeit und Bruchdehnung: Werstoff-Führer Kunststoffe, Hellerich, Harsch, Haenle,, 9.Auflage, Carl Hansen Verlag, 2004 S. 259-275).
In einer weiteren Ausführung der vorliegenden Erfindung handelt es sich bei dem Träger um einen Schwamm oder schwammartiges Material.
Unter Schwämmen sind in der Regel feste oder halbfeste elastische Schäume zu verstehen, die aus gasgefüllten beispielsweise polyederförmigen Zellen bestehen, die durch hochviskose und/oder feste Zellstege begrenzt sind. Einsetzbar sind sowohl natürlich vorkommende Schwämme, halbsynthetische oder synthetisch hergestellte schwammartige Gebilde. Beispiele für synthetische schwammartige Materialien sind Polyurethane, Polyacrylate, Poly(met)acrylsäurederivate, Homo- und Copolymere des Vinylacetats. Zu den natürlichen und halbsynthetischen Polymeren zählen u.a. Cellulose, Celluloseether oder Celluloseester wie Celluloseacetat und Celluloseacetatphthalat. Beispiele für natürliche Polymere sind Polysaccharide wie Alginate, Traganth, Xanthan Gumme, Guar Gummi und deren Salze und Derivate. Der Einsatz von Chitin und von Chitinderivaten ist möglich. Im Weiteren werden bevorzugt Stoffe mit Faserstruktur wie Skleroproteine z. B. Collagen, Keratin, Conchagene, Fibroin, Elastin und Chitin eingesetzt. Darüber hinaus sind auch stabil miteinander vernetzte Polysaccharide einsetzbar.

In einer weiteren Ausführung der vorliegenden Erfindung handelt es sich bei dem Träger um aus Textil, Vliesstoff, Papier und/oder Watte.

Die Materialien können gewebt, gestrickt oder gewirkt sein oder als Verbundstoff vorliegen. Zu den Verbundstoffen gehören nach der DIN 61210 T2 (außer Kraft) Vlies, Papier Watte und Filz, die alle erfindungsgemäß zu verwenden sind. Vliese sind lockere Materialien aus Spinnfasern (d.h. Faser mit begrenzter Länge) oder Filamenten (Endlosfasern), meist aus Polypropylen, Polyester oder Viskose hergestellt, deren Zusammenhalt im allgemeinen durch die den Fasern eigene Haftung gegeben ist. Hierbei können die Einzelfasern eine Vorzugsrichtung aufweisen (orientierte oder Kreuzlage-Vliese) oder ungerichtet (Wirrvliese) sein. Die Vliese können mechanisch verfestigt werden durch Vernadeln, Vermaschen oder durch Verwirbeln mittels scharfer Wasserstrahlen. Adhäsiv verfestigte Vliese entstehen durch Verkleben der Fasern mit flüssigen Bindemitteln (z.B. Acrylat-Polymere, SBR/NBR, Polyvinylester, Polyurethan-Dispersionen) oder durch Schmelzen oder Auflösen von sogenannten Bindefasern, die dem Vlies bei der Herstellung beigemischt wurden. Bei der kohäsiven Verfestigung werden die Faseroberflächen durch geeignete Chemikalien angelöst und durch Druck verbunden oder bei erhöhter Temperatur verschweisst
[J. Falbe, M. Regnitz: Römpp-Chemie-Lexikon, 9. Aufl. Thieme-Verlag, Stuttgart (1992)].

In einer weiteren Ausführung der vorliegenden Erfindung wird mit Watte eine lockere, aus Faserfloren aufgebaute und verdichtete Fasermasse bezeichnet, wobei die Fasern durch ihre natürliche Haftung zusammengehalten wird. Sie bestehen aus Baumwollhaaren, Wolle, Tillandsia, Zellstoff oder auch Quarz unter anderem Mineralfasern. Watte wird bevorzugt in Form von Wattepads oder Wattestäbchen oder in Kombinationen davon eingesetzt.

Unter Textilien sind im Rahmen der vorliegenden Erfindung Textilfasern, textile Halb-und Fertigfabrikate und daraus hergestellte Fertigwaren zu verstehen, die technischen Zwecken dienende textile Gebilde umfassen. Textilien können aus Materialien natürlichen Ursprungs sein, beispielsweise Baumwolle, Wolle oder Flachs, oder Mischgewebe, beispielsweise mit Baumwolle/Polyester, Baumwolle/Polyamid. Textilien können auch aus Polyacrylnitril, Polyamid und insbesondere Polyester bzw. Mischungen von Materialien natürlichen Ursprungs mit Polyacrylnitril, Polyamid und insbesondere Polyester bestehen.

Papier ist im Allgemeinen ein flächiger Werkstoff, der zu 60 bis 95 % aus mechanisch oder chemisch aufgeschlossenen Fasern meist pflanzlicher Herkunft besteht, die in einer wässrigen Suspension miteinander verbunden sind und unter Zusatz von Hilfsstoffen durch Entwässerung auf einem Sieb zur Blattform verfestigt werden. Papier besteht aus Fasern, Hilfsstoffen und Wasser. Neben pflanzlichen Fasern aus Zellstoff, Holzstoff (Holzschliff) und Hadern (z. B. Baumwolle, Hanffasern) verwendet man tierische, mineralische oder synthetische Fasern. Daneben stellt Altpapier als Sekundärfaserstoff mengenmäßig die wichtigste Faserstoffquelle für die Papierherstellung dar.

In einer besonderen Ausführungsform ist der erfindungsgemäße Träger ein Papier in Form von Tüchern wie ein Papiertaschentuch, Kosmetiktuch, Waschtuch, Handtuchpapier, Papierputztuch oder Küchentuch.

In einer weiteren Ausführungsform ist der Träger an einem Applikator befestigt. Dieser Applikator kann z.B. aus Kunststoff, Glas, Holz, Metall oder Textil sein. An dem Applikator kann ein Träger befestigt sein oder aber auch mehrerr gleiche Träger, z.B. zwei oder drei Schaumstoffe.

In einer weiteren Ausführungsform können an dem Applikator auch Kombinationen der Träger befestigt sein. In der folgenden Tabelle sind Träger T1 bis T68 genannt, die aus einer Kombination von jeweils mit einem "x" gekennzeichneten Material aufgebaut sind.

| Träger | Schaumstoffe | Schwamm | Textil | Vliesstoff | Papier | Watte |
|---|---|---|---|---|---|---|
| T1 | x | x | x | x | x | x |
| T2 | x | x | x | x | x | |
| T3 | x | x | x | x | | x |
| T4 | x | x | x | | x | x |
| T5 | x | x | | x | x | x |
| T6 | x | | x | x | x | x |
| T7 | | x | x | x | x | x |
| T8 | x | x | x | x | | |
| T9 | x | x | x | | x | |
| T10 | x | x | | x | x | |
| T11 | x | | x | x | x | |
| T12 | | x | x | x | x | |
| T13 | x | x | x | | | x |
| T14 | x | x | | x | | x |
| T15 | x | | x | x | | x |
| T16 | | x | x | x | | x |
| T17 | x | x | | | x | x |
| T18 | x | | x | | x | x |
| T19 | | x | x | | x | x |
| T20 | x | | | x | x | x |
| T21 | | x | | x | x | x |
| T22 | | | x | x | x | x |
| T23 | x | x | x | | | |
| T24 | x | x | | x | | |
| T25 | x | x | | | x | |
| T26 | x | x | | | | x |
| T27 | x | | x | x | | |
| T28 | | x | x | x | | |
| T29 | x | | x | | x | |
| T30 | x | | x | | | x |
| T31 | x | | | x | x | |
| T32 | x | | | x | | x |
| T33 | x | | | | x | x |
| T34 | | x | x | x | | |
| T35 | | x | x | | x | |
| T36 | | x | x | | | x |
| T37 | | x | | x | x | |
| T38 | | x | | x | | x |
| T39 | | x | | | x | x |
| T40 | | | x | x | | x |
| T41 | | | x | x | x | |
| T42 | | | x | | x | x |
| T43 | | | | x | x | x |
| T44 | x | x | | | | |
| T45 | x | | x | | | |
| T46 | x | | | x | | |
| T47 | x | | | | x | |
| T48 | x | | | | | x |
| T49 | | x | x | | | |
| T50 | | x | | x | | |
| T51 | | x | | | x | |
| T52 | | x | | | | x |
| T53 | | | x | x | | |
| T54 | | | x | | x | |
| T55 | | | x | | | x |
| T66 | | | | x | x | |
| T67 | | | | | x | x |
| T68 | | | | x | | x |

Der jeweilige Träger wird mit Hydrophobin beladen. Wobei es sich um ein Hydrophobin oder aber eine Mischung aus verschiedenen Hydrophobinen handeln kann, z.B. aus einer Mischung von zwei oder drei verschiedenen Hydrophobinen.

In einer Ausführung der vorliegenden Erfindung wird der Träger mit einer Mischung enthaltend Hydrophobin beladen.

Prinzipiell kann der träger auf jede Art mit Hydrophobin beladen werden. Der Fachmann kennt für die Beladung verschiedene Verfahren.
In einer Variante wird das sogenannte "Tauch-Verfahren" verwendet, bei dem der Träger in einem Tauchbad eingetaucht oder durch ein Bad gezogen wird.
Eine zweite Variante stellt das "Sprüh-Verfahren" dar, bei dem die Mischung auf den Träger aufgesprüht wird.
Als weitere Methode kommen sogenannte Abstreifmethoden zum Einsatz. Dort läuft der Träger z.B. als Vlies-, Papier-, Textil- oder Verbundbahnen an Abstreifblechen, - balken oder -düsen vorbei, die kontinuierlich mit der Hydrophobin enthaltenden Mischungen beladen werden.

In einer Ausführung der vorliegenden Erfindung wird zum Beladen des Trägers eine Mischung eingesetzt, welche Hydrophobin und mindestens Wasser oder ein wässriges Lösemittelgemisch umfasst.

Geeignete wässrige Lösemittelgemische umfassen Wasser sowie eines oder mehrere, mit Wasser mischbare Lösungsmittel. Die Auswahl derartiger Komponenten ist nur insofern beschränkt, als die Hydrophobine und die anderen Komponenten im Gemisch in ausreichendem Maße löslich sein müssen. Im Regelfalle umfassen derartige Gemische zumindest 50 Gew. %, bevorzugt mindestens 65 Gew. % und besonders bevorzugt mindestens 80 Gew. % Wasser. Ganz besonders bevorzugt wird nur Wasser eingesetzt. Der Fachmann trifft unter den mit Wasser mischbaren Lösungsmitteln je nach den gewünschten Eigenschaften der Mischung eine geeignete Auswahl. Beispiele geeigneter, mit Wasser mischbarer Lösungsmittel umfassen Monoalkohole wie Methanol, Ethanol oder Propanol, höhere Alkohole wie Ethylenglykol oder Polyetherpolyole sowie Etheralkohole wie Butylglykol oder Methoxypropanol.
Bevorzugt weist die zur Behandlung eingesetzte Mischung einen pH-Wert von 4 oder darüber, bevorzugt von 6 oder darüber und besonders bevorzugt von 7 oder darüber auf. Insbesondere liegt der pH-Wert im Bereich von 4 bis 11, bevorzugt von 6 bis 10, besonders bevorzugt von 7 bis 9,5 und ganz besonders bevorzugt von 7,5 bis 9. Beispielsweise kann der pH-Wert von 7,5 bis 8,5 oder von 8,5 bis 9 betragen.
Zur Einstellung des pH-Wertes umfasst die Mischung bevorzugt einen geeigneten Puffer. Der Fachmann wählt je nach dem zur Beladung vorgesehenen pH-Bereich einen geeigneten Puffer. Zu nennen sind beispielsweise Kaliumdihydrogenphosphat-Puffer, Tris(hydroxymethyl)aminomethan-Puffer (Tris-Puffer), Borax-Puffer, Natriumhydrogencarbonat-Puffer oder Natriumhydrogenphosphat-Puffer. Bevorzugt ist Tris-Puffer.
Die Konzentration des Puffers in der Lösung wird vom Fachmann je nach den gewünschten Eigenschaften der Mischung bestimmt. Der Fachmann wird in der Regel auf eine ausreichende Pufferkapazität achten, um möglichst gleichmäßige Beladung des Trägers zu erreichen. Bewährt hat sich eine Konzentration von 0,001 mol/l bis 1 mol/l, bevorzugt von 0,005 mol/l bis 0,1 mol/l und besonders bevorzugt von 0,01 mol/l bis 0,05 mol/l.
Die Konzentration der Hydrophobine in der Mischung wird vom Fachmann je nach den gewünschten Eigenschaften der Beladung gewählt. Mit höheren Konzentrationen lässt sich in der Regel eine schnellere Beladung erreichen. Bewährt hat sich im Regelfalle eine Konzentration von 0,1 µg/ml bis 1000 µg/ml, bevorzugt von 1µg/ml bis 500 µg/ml, besonders bevorzugt von 10 µg/ml bis 250 µg/ml, ganz besonders bevorzugt von 30 µg/ml bis 200 µg/ml und beispielsweise von 50 bis 100 µg/ml.

Die eingesetzte Mischung kann darüber hinaus optional weitere Komponenten umfassen. Beispiele zusätzlicher Komponenten umfassen Tenside. Geeignete Tenside sind beispielsweise nichtionische Tenside, welche Polyalkoxygruppen, insbesondere Polyethylenoxidgruppen umfassen. Beispiele umfassen Polyoxyethylenstearate, alkoxylierte Phenole und dergleichen. Weitere Beispiele geeigneter Tenside umfassen Polyethy-leneglycol(20)sorbitanmonolaurat (Tween® 20), Polyethyleneglycol(20)sorbitanmono-palmitat (Tween® 40), Polyethyleneglycol(20)sorbitanmonostearat (Tween® 60), Poly-ethyleneglycol(20)sorbitanmonooleat (Tween® 80), Cyclohexyl-methyl-β D-Maltosid, Cyclohexyl-ethyl-β D-Maltosid, Cyclohexyl-n-hexyl-β D-Maltosid, n-Undecyl-β D-Maltosid, n-Octyl-β D-Maltopyranosid, n-Octyl-β D-Glucopyranosid, n-Octyl-β D-Glucopyranosid, n-Dodecanoylsucrose. Weitere Tenside sind beispielsweise in WO 2005/68087 Seite 9, Zeile 10 bis Seite 10, Zeile 2 offenbart. Die Konzentration an Tensiden beträgt in der Regel von 0,001 bis 0,5 Gew. %, bevorzugt von 0,01 bis 0,25 Gew. % und besonders bevorzugt von 0,1 bis 0,2% Gew. %, jeweils bezogen auf die Menge aller Komponenten der Zubereitung.
Weiterhin können der Mischung noch Metallionen, insbesondere zweiwertige Metallionen zugegeben werden. Metallionen können zu einer gleichmäßigeren Beladung beitragen. Geeignete zweiwertiger Metallionen umfassen beispielsweise Erdalkalimetallionen wie Ca²⁺-lonen. Derartige Metallionen können bevorzugt als in der Formulierung lösliche Salze zugegeben werden, beispielsweise in Form von Chloriden, Nitraten oder Carbonat, Acetat, Citrat, Gluconat, Hydroxid, Lactat, Sulfat, Succinat, Tartrat. Beispielsweise können Calciumchlorid oder Magnesiumchlorid zugegeben werden. Die Löslichkeit kann optional auch durch geeignete Hilfsmittel, beispielsweise Komplexbildner gesteigert werden. Falls vorhanden, beträgt die Konzentration derartiger Metallionen in der Regel von 0,01 bis 10 mmol/l, bevorzugt von 0,1 bis 5 mmol/l und besonders bevorzugt von 0,5 bis 2 mmol/l.

In einer Ausführung der vorliegenden Erfindung sind die Träger zusätzlich zur Mischung enthaltend Hydrophobin mit Kosmetika beladen wie Gel, Schaum, Spray, einer Salbe, Creme, Emulsion, Suspension, Lotion, Milch oder Paste. Diese Kosmetika können einen weiteren positiven Effekt auf Haut und Haar bewirken.

Die Kosmetika können folgende Bestandteile enthalten:
- Farbstoffe z.B. solche, die dem Fachmann aus Handbüchern der Kosmetik bekannt sind
- kosmetisch und/oder dermatologisch aktive Wirkstoffe,z.B. färbende Wirkstoffe, Haut-und Haarpigmentierungsmittel, Tönungsmittel, Bräunungsmittel, Bleichmittel, keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon;
- kosmetische Mittel zur Pflege und zum Schutz der Haut, Nagelpflegemittel für die dekorative Kosmetik, hautkosmetische Mittel, z.B. Gesichtswässer, Gesichtsmasken und andere kosmetische Lotionen;

Die Zubereitungen können erhalten werden, indem die oben beschriebenen Hydrophobin enthaltenen Mischungen mit den gewünschten zusätzlichen oben genannten Komponenten gemischt und auf die gewünschte Konzentration verdünnt werden. Selbstverständlich können die Zubereitungen auch erhalten werden, indem man isolierte, feste Hydrophobine entsprechend löst.

Der Träger wird in jedem Fall mit der Hydrophobin enthaltenden Zubereitung behandelt. Um eine gleichmäßige Beladung des Trägers zu gewährleisten, sollte der Träger möglichst vollständig mit der Zubereitung getränkt werden. Die Beladung kann insbesondere vorgenommen werden, indem man den Träger in die Zubereitung eintaucht, mit der Zubereitung besprüht oder mit der Zubereitung begießt.

In der Regel ist eine gewisse Einwirkzeit erforderlich, um die Träger zu beladen. Der Fachmann wählt je nach dem gewünschten Ergebnis eine geeignete Einwirkzeit. Beispiele typischer Einwirkzeiten liegen in einem Zeitraum von 0,1 bis 12 h, ohne dass die Erfindung hierauf beschränkt sein soll. Sie kann z.B. durch Anlegen von Druck oder Vakuum beeinflusst werden.

Im Regelfalle ist die Einwirkzeit von der Temperatur sowie von der Konzentration des Hydrophobins in der Mischung bzw. Zubereitung abhängig. Je höher die Temperatur und je höher die Konzentration im Zuge des Beladungsvorganges, desto kürzer kann die Einwirkzeit sein. Die Temperatur im Zuge des Beladungsvorganges kann bei Raumtemperatur liegen oder aber es kann sich um erhöhte Temperaturen handeln. Beispielsweise kann es sich um Temperaturen von 5, 10, 20, 30, 40, 50, 60, 70, 80, 90, 100, 110, oder 120°C handeln. Bevorzugt handelt es sich um Temperaturen von 15 bis 120°C, besonders bevorzugt von 20 bis 100°C, und beispielsweise von 40 bis 100°C oder von 70 bis 90°C. Die Temperatur kann beispielsweise durch Erwärmen des Bades, in welches der zu beladende Träger eingetaucht wird, eingebracht werden. Man kann aber auch einen eingetauchten Träger nachträglich erwärmen, beispielsweise mit Hilfe von IR-Strahlern.

In einer bevorzugten Ausführungsform der Erfindung erfolgt die Beladung mit Hydrophobin in Gegenwart von Mikrowellenbestrahlung. Dadurch kann die Einwirkzeit sehr stark reduziert werden. Je nach Energieeintrag reichen unter Umständen bereits wenige Sekunden zur Beladung des Trägers mit Hydrophobin aus.

In einer bevorzugten Ausführungsform wird nach dem Beladen das Lösungsmittel aus dem Träger entfernt. Bevorzugt kann das Lösungsmittel mechanisch durch Ausüben von Druck aus dem Träger entfernt werden. Optional kann der Träger zuvor noch mit Wasser oder einem bevorzugt wässrigen Lösungsmittelgemisch gewaschen werden. Das Entfernen des Lösungsmittels kann beispielsweise durch einfaches Abdampfen an Luft erfolgen. Das Entfernen des Lösungsmittels kann aber auch durch Erhöhen der Temperatur und/oder mit geeigneten Gasströmen und/oder Anlegen eines Vakuums erleichtert werden. Das Abdampfen kann erleichtert werden, indem man beispielsweise beladene Träger in einem Trockenschrank erwärmt oder mit einem erwärmten Gasstrom anbläst. Die Methoden können auch kombiniert werden, beispielsweise indem man in einem Umlufttrockenschrank oder einem Trockenkanal trocknet. Weiterhin kann der beladene Träger zum Entfernen des Lösungsmittels auch mittels Strahlung, insbesondere IR-Stahlung erwärmt werden. Hierzu können alle Arten von breitbandigen IR-Strahlern, bspw. NIR-, MIR- oder NIR-Stahler eingesetzt werden. Es können aber beispielsweise auch IR-Laser eingesetzt werden. Derartige Strahlungsquellen sind in diversen Strahlungsgeometrien kommerziell erhältlich.

Die Temperatur sowie die Trockenzeit im Zuge des Trocknens wird vom Fachmann festgelegt. Bewährt hat sich im Allgemeinen eine Trocknungstemperatur von 30 bis 130°C, bevorzugt von 50 bis 120°C, besonders bevorzugt von 70 bis 110°C, ganz besonders bevorzugt von 75 bis 105°C und beispielsweise von 85 bis 100°C. Gemeint ist hierbei die Temperatur des Trägers selbst. Die Temperatur in einem Trockner kann selbstverständlich auch höher sein. Die Trockenzeit ist naturgemäß umso kürzer, je höher die Trockentemperatur ist. Ebenso können die Trocknungstemperaturen niedriger sowie die Trocknungszeit kürzer sein, sofern die Trocknung bei Unterdruck vorgenommen wird.

Die Temperaturbehandlung im Zuge des Beladens des Trägers und die Trocknung können vorteilhaft miteinander kombiniert werden. So kann beispielsweise ein Träger zunächst mit der Mischung bzw. Zubereitung bei Raumtemperatur behandelt und anschließend bei erhöhten Temperaturen getrocknet und getempert werden. In einer bevorzugten Ausführungsform des Verfahrens wird mindestens in einem der beiden Schritte "Beladung" oder "Trocknung" erhöhte Temperatur angewandt. Bevorzugt wird in beiden Schritten höhere Temperatur als Raumtemperatur angewandt.

Die Beladung kann unmittelbar im Anschluss an den Herstellprozess des Trägers vorgenommen werden, beispielsweise durch den Hersteller des Trägers selbst. Sie kann aber selbstverständlich auch erst zu einem späteren Zeitpunkt vorgenommen werden, beispielsweise durch einen Weiterverarbeiter oder nach dem Ausliefern des Trägers an den Endverbraucher von diesem selbst.

In einer zweiten Ausführungsform der Erfindung können die beladenen Träger erhalten werden, indem man die Herstellung des Trägers in Gegenwart von Hydrophobin vornimmt

In einer weiteren Ausführungsform der Erfindung können die beladenen Schaumstoffe erhalten werden, indem man die Herstellung des Schaumstoffes in Gegenwart von Hydrophobin vornimmt.

Im Falle der Herstellung von offenzelligen Schaumstoffen auf Basis von Melamin-Formaldehyd-Kondensationsprodukten kann hierzu das Hydrophobin sowie optional weitere der oben genannten Bestandteile mit der oben erwähnten wässrigen Lösung oder Dispersion des Melamin-Formaldehyd-Vorkondensates, eines Treibmittels, eines Dispergiermittels sowie eines Härters vermischt werden. Anschließend kann die Mischung in prinzipiell bekannter Art und Weise erwärmt, verschäumt und ausgehärtet werden.

In einer bevorzugten Ausführungsform ist der erfindungsgemäße Träger trocken.

Von besonderem Vorteil für die erfindungsgemäßen Träger sowie für das erfindungsgemäße Verfahren ist, dass das Hydrophobin auf den Träger auch im trockenen Zustand stabil verbleibt.

Die erfindungsgemäßen Träger werden bevorzugt eingesetzt in einem Verfahren zum Entfernen von wasserunlöslichen Substanzen.

In der Regel handelt es sich dabei um mehrere Substanzen, es kann aber auch nur um eine Substanz handeln.

Die Wasserlöslichkeit der Substanzen, die erfindungsgemäß von Substratoberflächen entfernt werden, kann von im Wesentlichen wasserunlöslich bis gänzlich wasserunlöslich reichen. Im Allgemeinen beträgt die Löslichkeit der Substanzen in Wasser etwa 1 g/l bei Raumtemperatur. Darüber hinaus kann die Löslichkeit in Wasser bei Raumtemperatur aber geringer sein als 1 g/l.

In einer Ausführung der vorliegenden Erfindung sind die wasserunlöslichen Substanzen Fette, Öle, Wachse und/oder Zusammensetzungen enthaltend Fette, Wachse und/oder Öle. Zusammensetzungen können außer Ölen, Fetten und/oder Wachsen auch noch weitere Substanzen enthalten.

In einer Ausführungsform können die Zusammensetzungen auch aus einer Kombination aus Ölen, Fetten und Wachsen umfassen:
a) Öle, Wachse und Fette
b) Öle und Wachse
c) Öle und Fette
d) Wachse und Fette

In einer Ausführung der vorliegenden Erfindung sind Öle wasserunlösliche, flüssige organische Verbindungen mit relativ niedrigem Dampfdruck. Zu den Ölen zählen fette Öle, ätherische Öle, Mineralöle und Silikonöle.
Fette Öle sind Fette, also Gemische von Fettsäuretriglyceriden, die bei Raumtemperatur flüssig sind, während Fette bei Raumtemperatur fest sind.

Ätherische Öle sind ölige, wasserdampf-flüchtige Extrakte aus Pflanzen oder Pflanzenteilen, die abhängig von der Herkunftspflanze einen starken, charakteristischen Geruch haben. Sie bestehen größtenteils aus Terpenen. (Beispiel: Zitronenöl). Ätherische Öle können aber auch künstlich hergestellt werden.

Mineralöle werden aus Erdölen oder Kohlen gewonnen und sind Kohlenwasserstoffverbindungen. Die meisten Verbindungen in den Stoffgemischen gehören chemisch gesehen zu den Alkanen.

Silikonöle basieren auf Polymeren und Copolymeren aus Silizium-Sauerstoff-Einheiten und organischen Seitenketten. Sie sind relativ unempfindlich gegenüber Oxidation, Wärme und anderen Einflüssen.

In einer Ausführung der vorliegenden Erfindung sind Fette Ester des dreiwertigen Alkohols Glycerin (Propan-1,2,3-triol) mit drei, meist verschiedenen, überwiegend geradzahligen und unverzweigten aliphatischen Monocarbonsäuren, den so genannten Fettsäuren.

In einer Ausführung der vorliegenden Erfindung sind Wachse Stoffe, die heute durch ihre mechanisch-physikalischen Eigenschaften definiert werden. Ihre chemischen Zusammensetzung und Herkunft sind hingegen sehr unterschiedlich. Ein Stoff wird als Wachs bezeichnet, wenn er bei 20 °C knetbar, fest bis brüchig hart ist, eine grobe bis feinkristalline Struktur aufweist, farblich durchscheinend bis opak, aber nicht glasartig ist, über 40 °C ohne Zersetzung schmilzt, wenig oberhalb des Schmelzpunktes leicht flüssig (wenig viskos) ist, eine stark temperaturabhängige Konsistenz und Löslichkeit aufweist sowie unter leichtem Druck polierbar ist.

Tierische und pflanzliche Wachse zählen zu den Lipiden. Hauptkomponenten dieser Stoffgemische sind Ester von Fettsäuren mit langkettigen, aliphatischen, primären Alkoholen. Myricin ist z.B. ein Ester der Palmitinsäure mit Myristylalkohol und der Haupbestandteil von Bienenwachs. Diese Ester unterscheiden sich in ihrem Aufbau von den Fetten, die Triglyceride von Fettsäuren sind.

Tierische Wachse sind beispielsweise Walrat und Bienenwachs. Pflanzliche Wachse sind beispielsweise Zuckerrohrwachs, Carnaubawachs der Wachspalme. Das Jojobaöl ist kein Triglycerid und damit eigentlich kein Öl, sondern chemisch betrachtet ein flüssiges Wachs.

Geologische Erdwachse (Ozokerit und das daraus hergestellte Ceresin) bestehen im Wesentlichen aus Kohlenwasserstoffen.

Synthetische Wachse werden hauptsächlich aus Erdöl gewonnen. Hauptprodukt ist Hartparaffin, das z.B für Kerzen oder Schuhcreme verwendet wird. Für spezielle Anwendungen werden natürliche Wachse chemisch modifiziert oder vollständig synthetisiert (Polyethylene, Copolymere). Auch aus Soja kann durch Hydrierung Sojawachs hergestellt werden.

In einer Ausführung der vorliegenden Erfindung sind die wasserunlöslichen Substanzen Sebum und/oder Schminke.

In einer Ausführung der vorliegenden Erfindung ist unter Schminke dekorative Kosmetika oder Make Up zu verstehen.

Dekorative Kosmetika sind Substanzen, die zur einmaligen Verwendung auf (Gesichts-) Haut, Lippen, Haar oder Nägel aufgetragen werden, zum ausschließlichen oder überwiegenden Zweck, deren Aussehen innerhalb kurzer Zeit deutlich, meist farblich, aber reversibel zu verändern.

Im Allgemeinen ist Sebum ein Hautoberflächenfett, das zum geringeren Teil aus dem Hornfett, einem Nebenprodukt der Keratinisierung, besteht und aus den Talgdrüsen stammt. Die durchschnittliche Zusammensetung des Sebums besteht aus: Triglyceride (19,5 - 49,4 % Massengehalt), Wachsester (22,6 - 29,5 % Massengehalt), Fettsäuren (7,9 - 39,0 % Massengehalt), Squalen (10,1 - 13,9 % Massengehalt), Diglyceride (1,3 - 4,3 % Massengehalt), Cholesterinester (1,5 - 2,6 % Massengehalt), Cholesterin (1,2 - 2,3 % Massengehalt).

In einer Ausführung der vorliegenden Erfindung wird ein Träger enthaltend Hydrophobin in einem kosmetischen Verfahren verwendet, um Sebum von Haut zu entfernen. In einer weiteren Ausführung der vorliegenden Erfindung wird ein Träger enthaltend Hydrophobin in einem kosmetischen Verfahren verwendet, um Sebum von Haar zu entfernen. In einer weiteren Ausführung der vorliegenden Erfindung wird ein Träger enthaltend Hydrophobin in einem kosmetischen Verfahren verwendet, um Schminke von Haut zu entfernen. In einer weiteren Ausführung der vorliegenden Erfindung wird ein Träger enthaltend Hydrophobin in einem kosmetischen Verfahren verwendet, um Schminke von Haar zu entfernen. In einer weiteren Ausführung der vorliegenden Erfindung wird ein Träger enthaltend Hydrophobin in einem kosmetischen Verfahren verwendet, um Schminke und Sebum von Haut zu entfernen. In einer weiteren Ausführung der vorliegenden Erfindung wird ein Träger enthaltend Hydrophobin in einem kosmetischen Verfahren verwendet, um Schminke und von Haar zu entfernen.

In dem erfindungsgemäßen Verfahren werden wasserunlösliche Substanzen von Substratoberflächen entfernt.

Als Substrate können Feststoffe dienen. Die Feststoffe können amorph, kristallin oder teilkristallin sein. Ein Substrat kann aus einem oder mehreren Feststoffen aufgebaut sein. Es ist möglich, dass ein Substrat Gas- und/ oder Flüssigkeitseinschlüsse umfasst.

In einer Ausführung der vorliegenden Erfindung bestehen die Substrate aus Glas, Kunststoff, Holz, Metall, Textil, Federn, Fell, Haut und/oder Haar.

Glas kann technische Gläser, optische Gläser und dekorative Gläser umfassen. Beispiele hierfür sind Flachgläser wie Fensterscheiben und Spiegel.

Im Allgemeinen ist ein Kunststoff ein Festkörper, dessen Grundbestandteil synthetisch oder halbsynthetisch erzeugte Polymere mit organischen Gruppen sind. Kunststoffe werden in sehr vielen Bereichen eingesetzt, wie zum Beispiel im Automobilbau, Bauindustrie, Haushalt und Garten.

Im Allgemeinen enthält Holz lignifiziertes (verholztes) pflanzliches Gewebe. In einer Ausführungsform wird unter Holz auch beschichtetes Holz verstanden wie es zum Beispiel bei der Herstellung von Möbeln, Parkett und ähnlichen Bodenbelägen und Spielzeug verwendet wird.

Im Allgemeinen sind unter dem Begriff Metalle alle chemischen Elemente außer Wasserstoff, Kohlenstoff, Phosphor, Selen, Jod, Radon, Helium, Neon, Argon, Krypton, Xenon, Radon und Ununoctium zu verstehen. Ferner fällt unter den Begriff Metall auch Edelstahl zum Beispiel von Haushaltsgeräten oder Besteck. Edelstahl (nach DIN EN 10020) ist eine Bezeichnung für legierte oder unlegierte Stähle mit besonderem Reinheitsgrad, zum Beispiel Stähle, deren Schwefel- und Phosphorgehalt (sog. Eisenbegleiter) 0,025 % nicht übersteigt.

Ferner fallen unter den Begriff auch Metalllegierungen.

In einer Ausführung der vorliegenden Erfindung betrifft der Begriff Fell das tierische Fell. In einer Ausführung der vorliegenden Erfindung betrifft der Begriff Feder die tierische Feder.

In einer Ausführung der vorliegenden Erfindung betrifft der Begriff Haut die menschliche Gesichtshaut.

Die Substratoberfläche ist in erster Linie die äußere Schicht des zu behandelnden Substrats. Die Substratoberfläche kann glatt, eben, nicht gänzlich glatt, sondern unregelmäßig sein, beispielsweise kann jene Oberfläche Vertiefungen aufweisen. Die Substratoberfläche kann auch Hohlräume, Zellen und/oder Poren aufweisen.

Das erfindungsgemäße Verfahren zeichnet sich durch die einfache und leichte Handhabung der Hydrophobin enthaltenden Träger aus. Der mit Hydrophobin beladene Träger entfernt durch einfaches Kontaktieren der Substratoberfläche z.B. in Form von Wischen oder Tupfen mit leichtem Druck die wasserunlöslichen Substanzen.

Durch das erfindungsgemäße Verfahren wird gewährleistet, dass mittels eines Trägers schonend wasserunlösliche Substanzen entfernt werden.

In einer Ausführungsform wird Hydrophobin zur Herstellung eines kosmetischen Mittels zum Entfernen von Sebum verwendet.

In einer weiteren Ausführungsform wird Hydrophobin zur Herstellung eines kosmetischen Mittels zur Prophylaxe von Akne verwendet.

Im Allgemeinen sind kosmetische Mittel Stoffe oder Zubereitungen aus Stoffen, die ausschließlich oder überwiegend dazu bestimmt sind, äußerlich am Körper des Menschen oder in seiner Mundhöhle zur Reinigung, zum Schutz, zur Erhaltung eines guten Zustandes, zur Parfümierung, zur Veränderung des Aussehens oder dazu angewendet zu werden, den Körpergeruch zu beeinflussen. Der Begriff "kosmetische Mittel" umfasst also nicht nur dekorative Kosmetika, sondern auch pflegende Mittel wie Hautcremes oder Mittel zur Körperreinigung wie Zahnpasten, Seifen, Haarshampoos etc.
Die Akne ist eine Erkrankung der Talgdrüsenfollikel (folliculus = Schlauch), die in den Haarkanal münden. Das in den Talgdrüsen produzierte Sebum fettet Haut und Haar, bildet einen Gleitfilm im Haarkanal und dichtet den Haarkanal nach außen ab. Im Bereich des Haaraustrittes ist die Hornschicht dünner und nach innen gewölbt. Mit dem austretenden Sebum werden daher auch abgelöste Hornschichtzellen nach oben transportiert.

In einer weiteren Ausführungsform wird Hydrophobin zur Herstellung eines kosmetischen Mittels zur Prophylaxe von Keratosis pilaris verwendet.

In einer weiteren Ausführungsform wird Hydrophobin zur Herstellung eines kosmetischen Mittels zur Prophylaxe von Akne und Keratosis pilaris verwendet.

Der Vorteil der vorliegenden Erfindung liegt in einem Verfahren zum Entfernen von wasserunlöslichen Substanzen von Substratoberflächen zur Verfügung, das vielseitig einsetzbar ist. Darüber hinaus ist das Verfahren schonend für die Substratoberfläche. Zusätzlich ist das Verfahren einfach und erlaubt das Entfernen der wasserunlöslichen Substanzen mit wenigen Handgriffen.

### Beispiel 1:

Entfernen von Sebum mittels eines mit Hydrophobin beladenen Schaumstoffs. Würfelförmige Proben (2,5cm x 2,5 cm x 2,5 cm) eines offenzelligen Melamin-Formaldehyd-Schaumstoffs mit einer Dichte von 9 kg/m³ (Basotect ®, BASF Aktiengesellschaft) wurden mit einer Lösung von 0,1 g/l Hydrophobin A (SEQ ID 20 aus WO2007/14897) bzw. Hydrophobin B (SEQ ID 26 aus WO2007/14897) getränkt. Die Lösung mit dem getränkten Schaumstoffwürfel wurde 15 h bei 60°C erwärmt. Anschließend wurde die wässrige Lösung abdekantiert. Die Schaumstoffwürfel wurden durch Auspressen vom Großteil der aufgenommenen Flüssigkeit befreit, mehrmals mit Reinstwasser gewaschen und ausgepresst und bei 40°C bis zur Gewichtskonstanz getrocknet.

Das Entfernen des natürlichen Sebums wurde auf unbehandelter humaner Haut, im Stirnbereich getestet. Kleine zugeschnittene Würfel (Kantenlänge ca. 2,5 cm) wurden mit leichtem Druck über die Haut geführt.

Bestimmung des verbleibenden natürlichen Sebums auf der Haut wurde mit dem Sebumeter SM81 0 der Firma Courage + Khazaka Electronic GmbH ausgewertet. Vor der Messung muß ein Nullabgleich des Gerätes auf die Folie zur Fettmessung nach Angaben des Herstellers durchgeführt werden.

**Tabelle der Ergebnisse**

| Nr. | Probe | Verbleibendes natürliches Sebum (%) |
|---|---|---|
| K | Kontrolle (Schwamm ohne Hydrophobin) | 100 |
| V1 | Schwamm mit Hydrophobin B | 21 |
| V2 | Schwamm mit Hydrophobin A | 28 |

"K" bezeichnet hierbei den Kontrollversuch und "V" den erfindungsgemäßen Vergleichsversuch.

Die beladenen Schaumstoffwürfel zeigten ein verbessertes Entfernen von natürlichem Sebum, gemessen auf unbehandelter humaner Haut.

### Beispiel 2:

Hydrophobin beladener Schaumstoffwürfel zur Aufnahme von künstlichem Sebum

Für den Versuch wurde 20 g künstliches Sebum (Zusammensetzung siehe unten) in ein Urglas gegeben und ein Schaumstoffwürfel eines offenzelligen Melamin-Formaldehyd-Schaumstoffs mit einer Dichte von 9 kg/m³ (Basotect ®, BASF Aktiengesellschaft) mit einer Kantenlänge von 15 x 15 mm für 1 min hineingelegt. Dabei wurde ein unbehandelter Schaumstoffwürfelwürfel, ein mit Hydrophobin B beladener Schaumstoffwürfelwürfe und ein mit Casein "beladener" Schaumstoffwürfelwürfel (analog zur Beladung von Hydrophobin B, siehe Beispiel 1) verglichen.

Ebenso wurde der Versuch auch mit reinem Capric/Caprylic Triglycerid (Miglyol^{®} 812, Firma Sasol) durchgeführt.

Die aufgenommene Menge Sebum/Triglycerid wurde mittels Analysenwaage ermittelt.

Es wurde die Sebum-/ Triglyceridaufnahme im Verhältnis zum Gewicht des Schaumstoffwürfelwürfels berechnet. Anschließend wurden die Schaumstoffwürfelwürfel für 30 min auf Filterpapier (Pörringer Typ 1243/90) gelegt. Das Gewicht wurde erneut mit der Analysenwaage ermittelt und die Sebumabgabe berechnet.

Zusätzlich wurden die Würfel auf Wasser gelegt und ihr Verhalten beobachtet.

### Ergebnis :

| Nr. | Träger: Schaumstoffwürfel | Sebumaufnahme in % | Sebumabgabe in % |
|---|---|---|---|
| V3 | mit Hydrophobin B beladen | 74,5 | 3 |
| K2 | unbehandelt | 60,5 | 4 |
| K3 | mit Casein beladen | 39,8 | 5 |

Es zeigte sich, dass der erfindungsgemäße Träger (V3) die größte Menge künstliches Sebum aufnehmen konnte und anschließend am wenigsten künstliches Sebum abgab. Ein mit einem willkürlich gewählten Vergleichsprotein (Casein) beladener Träger zeigte eine deutlich verringerte Sebumaufnahme, sowie eine höhere anschließende Sebumabgabe.

### Zusammensetzung des künstlichen Sebums :

| | |
|---|---|
| 41,0% | Triglyceride |
| 25,0% | Wachsester |
| 16,0% | Fettsäuren |
| 13,0% | Squalen |
| 1,0% | Diglyceride |
| 2,0% | Cholesterinester |
| 2,0% | Cholesterin |

### Beispiel 3:

### Beladung von Wattepads mit Hydrophobin B

### Herstellen der Lösungen:

Hydrophobin B: Das Hydrophobin B (Granulat oder Pulver) wurde zunächst in Wasser bei Raumtemperatur vorgelöst. Die maximale Löslichkeit lag bei 50mg/mL (= 5%). Zur Beschleunigung wurde die Lösung auf 60°C erhitzt. Nach der vollständigen Lösung wurde der Proteingehalt mittels Bradford bestimmt.
Beladungspuffer: Beim Beladungspuffer handelte es sich um einen 50mM Tris-Puffer. Es wurden 1 mM CaCl₂ zugegeben und der pH-Wert mit 32% HCl auf 8 eingestellt.

### Ansetzen der Beladung:

Der Puffer wurde in ein Becherglas gefüllt und das vorgelöste Hydrophobin B bis zu einer Endkonzentration von 50µg/mL zugegeben und verrührt.
Die Wattepads wurden in das Becherglas gegeben. Sie wurden vollständig in die Lösung eintaucht.

### Inkubation:

Das Becherglas wurde abgedeckt und dann für 16h in einem Wärmeschrank bei 50°C inkubiert.

### Waschen:

Anschließend wurden die Pads unter fließendem VE-Wasser gründlich ausgewaschen.

### Trocknen:

Die Wattepads wurden für 24h bei 50°C im Wärmeschrank getrocknet.

### Aktivität:

Anschließend konnte der Erfolg der Beladung überprüft werden, in dem man einen Wassertropfen (ca. 50µL) auf das Wattepad gab. Blieb der Tropfen auf dem mit Hydrophobin behandelten Pad im Vergleich zu einem unbehandelten Pad länger auf der Oberfläche stehen (ca. 5 Sekunden) anstatt einzuziehen, wurde der beladene Wattepad weiter eingesetzt.

### Beispiel 4:

### Hydrophobin beladene Wattepads zur Aufnahme von künstlichem Sebum

### 1. Versuch:

Für den Versuch wurde 20 g künstliches Sebum (Zusammensetzung siehe Beispiel 2) bzw. in einer Versuchsvariante Capric/Caprylic Triglycerid (Miglyol^{®} 812, Firma Sasol) in ein Urglas gegeben und die unbehandelten und mit Hydrophbin B beladenen Wattepads 1 min hineingelegt (Beladung siehe Beispiel 3).

Die Wattepads wurden mit einem Gewicht beschwert und anschließend in ein Gefäß mit Wasser gelegt.

### 2. Versuch:

Auf die unbehandelten und mit Hydrophobin B beladenen Wattepads wurde ein Tropfen Wasser gegeben und beobachtet, wie schnell der Tropfen einsinkt (Beladung siehe Beispiel 3).

### Ergebnis :

### zu Versuch 1:

Das unbehandelte Wattepad gab das Sebum / Triglycerid schnell wieder ab. Das Wattepad quoll stark auf.
Das mit Hydrophobin B behandelte Wattepad hielt das Sebum / Triglycerid deutlich besser und blieb auch formstabil.

### zu Versuch 2:

Bei unbehandelten Wattepads sank der Tropfen sofort ein, während das mit Hydrophobin B behandelte Wattepad den Tropfen deutlich länger auf der Oberfläche hielt (Abperleffekt).

## Patentansprüche

1. Verfahren zum Entfernen von wasserunlöslichen Substanzen von Substratoberflächen mittels eines Trägers enthaltend Hydrophobin.

2. Verfahren nach Anspruch 1 **dadurch gekennzeichnet, daß** die wasserunlöslichen Substanzen Fette, Öle, Wachse oder Zusammensetzungen enthaltend Fette, Wachse und/oder Öle sind.

3. Verfahren nach einem der Ansprüche 1 bis 2 **dadurch gekennzeichnet, daß** die wasserunlöslichen Substanzen Sebum und/oder Schminke sind.

4. Verfahren nach mindestens einem der Ansprüche 1 bis 3 **dadurch gekennzeichnet, dass** die Substrate: Glas, Kunststoff, Holz, Metall, Textil, Haut und/oder Haar sind.

5. Verfahren nach mindestens einem der Ansprüche 1 bis 4 **dadurch gekennzeichnet, dass** der Träger aus Schaumstoff, Schwamm, Textil, Vliesstoff, Papier und/oder Watte ist.

6. Kosmetisches Verfahren zum Entfernen von Sebum und/oder Schminke **dadurch gekennzeichnet, dass** die Haut- und/oder Haaroberflächen mit einem Träger enthaltend Hydrophobin behandelt werden.

7. Verwendung eines Trägers enthaltend Hydrophobin zum Entfernen von wasserunlöslichen Substanzen von Substratoberflächen.

8. Verwendung nach Anspruch 7 **dadurch gekennzeichnet, dass** die wasserunlöslichen Substanzen Fette, Öle, Wachse oder Zusammensetzungen enthaltend Fette, Wachse und/oder Öle sind.

9. Verwendung nach einem der Ansprüche 7 bis 8 **dadurch gekennzeichnet, daß** die wasserunlöslichen Substanzen Sebum und/oder Schminke sind.

10. Verwendung nach mindestens einem der Ansprüche 7 bis 9 **dadurch gekennzeichnet, dass** die Substrate Glas, Kunststoff, Holz, Metall, Textil Haut und/oder Haar sind.

11. Verwendung nach mindestens einem der Ansprüche 7 bis 10 **dadurch gekennzeichnet, dass** der Träger aus Schaumstoff, Schwamm, Textil, Vliesstoff, Papier und/oder Watte ist.

12. Verwendung eines Trägers enthaltend Hydrophobin in einem kosmetischen Verfahren zum Entfernen von Sebum und/oder Schminke von Haut- und/oder Haaroberflächen.

13. Verwendung von Hydrophobin zur Herstellung eines kosmetisches Mittels zum Entfernen von Sebum.

14. Verwendung von Hydrophobin zur Herstellung eines kosmetischen Mittels zur Prophylaxe und/oder Behandlung von Akne und/oder Keratosis Pilaris.
